# EUROPEAN PATENT SPECIFICATION

(11) **EP 3 085 311 B1**
(45) Date of publication and mention of the grant of the patent: **26.12.2018**
(21) Application number: 16166028.7
(22) Date of filing: 19.04.2016
(51) Int. Cl.: A61B 17/04, A61B 17/88, A61B 17/00, A61B 90/00, A61B 17/34

(54) **INDICATOR FOR INSTALLING A MEDICAL DEVICE**
INDIKATOR ZUR INSTALLATION EINER MEDIZINISCHEN VORRICHTUNG
INDICATEUR POUR L'INSTALLATION D'UN DISPOSITIF MEDICAL

(30) Priority: 21.04.2015 EP 15164478
(43) Date of publication of application: 26.10.2016
(73) Proprietor: Oticon Medical A/S, 2765 Smørum (DK)
(72) Inventor: Johansson, Martin, DK-2765 Smørum (DK); Holmberg, Marcus, DK-2765 Smørum (DK); Jonhede, Sofia, DK-2765 Smørum (DK); Jonsson, Claes, DK-2765 Smørum (DK)
(74) Representative: Nielsen, Hans Jørgen Vind

(56) References cited:
- CN-A- 103 567 938
- US-A1- 2002 120 292
- US-A1- 2008 319 570
- US-A1- 2009 187 194
- US-A1- 2013 178 855

## Description

### FIELD

The disclosure relates to installing a medical device. In particular, the disclosure relates to an indicator unit configured to indicate a length that the medical device traverses during an installation process and to indicate proper seating of the medical device in a recipient of the medical device. The indicator unit may also be used to aid an operator during installation for installing the medical device (implant) in alignment with a predrilled hole so that the implant is inserted perpendicular to a skin surface and/ or bone surface. The indicator unit is used in combination with an inserter that is adapted to install the medical device. In an illustrative disclosure, the indicator unit is disclosed in relation to a bone anchored hearing aid.

### BACKGROUND

For persons who cannot benefit from traditional, air conduction hearing aids there are other types of sound transmitting hearing aids, i.e. bone anchored hearing aids. In bone anchored hearing aids, a speech processor comprising microphone(s) and a vibrator is connected to an implant anchored to the skull. The speech processor captures and processes the sound received at the microphone(s) and the processed sound information is then mechanically transmitted using the vibrator via the bone anchored implant through the skull bone to a person's inner ear. The bone anchoring principle indicates that the skin is penetrated through a surgical process and the implant is installed in the bone. This makes the vibratory transmission very efficient.

The implants that are used today are normally in two pieces - one piece includes the screw-shaped anchoring element (fixture) that attaches to a skull bone of the user, and the other piece includes a skin penetrating abutment that is detachably connected to the anchoring element. The two-piece design allows for upgrading, if necessary, without removing the fixture, and if the abutment sleeve is damaged then it may also be replaced without need of removal of the bone anchored fixture.

During the installation process, it is critical that that the implant is properly seated, i.e. that the implant is fully inserted and that the implant is inserted perpendicular to the skin surface and/ or bone surface. If the implant is not fully inserted, then the torque required for installation may increase for example up to 50 Ncm and in some case may even require manual insertion.

Current method of verification of whether the implant is seated properly include visual inspection. Such inspection is generally possible when performing a conventional flap surgery, i.e. surgery involving fully dissecting soft tissue around an installation site and uncovering a bone surface where implant is to be installed. However, when performing minimally invasive surgery, i.e. where the bone surface remains covered by soft tissue such as disclosed in Pending EP applications numbered EP13197428.9, EP14190289.0 and EP15157597.4 such visual confirmation of seating of the implant is not possible. Therefore, there is a need of an alternate solution that would allow verifying proper seating of the implant.

US2002/120292 relates to a suture anchor for securing soft tissue to bone including a body extending along a longitudinal axis between opposite ends, and at least one eyelet extending between lateral surfaces of the body for receiving a suture. The eyelet defines two entry/exit void regions and a central void region extending between the entry/exit void regions. Each entry/exit void region extends from one of the lateral surfaces of the body and has an axis forming an acute angle with the longitudinal axis of the body. Each entry/exit void region also has a surface comprising a segment of a conic surface extending about the axis of the entry/exit void region and tapering inward towards the longitudinal axis of the body. The central void region extends along an axis transverse to the longitudinal axis of the body.

US2009/187194 relates to a one step entry pedicular preparation device works well with Minimal Invasive Spine Surgery (MISS) to facilitating such approach. A related intervertebral disc access system and pedicle screw compatible with the systems is also illustrated. The systems include a manipulator having a bar handle and main body with main barrel bore a pedicle dart having a proximal tip end and a second distal open end selectably attachable to the manipulator using a variety of interconnect configurations, both of which work in conjunction with a guide pin. The pedicle darts can be made of any material, disposable or re-usable and can be utilized for a variety of purposes, including bone structure formation for faster conventional pedicle screw insertion with precision and vertebra fixation.

During the installation, it is also important to have the implant installed in alignment with a pre-drilled hole available in the bone. Not doing so, may result in the implant getting stuck midway even if sufficient torque is applied. Furthermore, a misaligned installation results in a tilted implant that exerts tension on the skin. This could impair healing of soft tissue surrounding the abutment and causes micro-traumata at the skin-implant interface. Therefore, it is also desirable to have the implant installed in alignment of the hole i.e. that the center axis of the implant/abutment is aligned with the center axis of the hole.

### SUMMARY

According to an embodiment, an inserter for installing a medical device via rotation in a recipient is disclosed. The inserter includes a longitudinal member adapted to rotate around a member axis, the longitudinal member comprising a first end and a second end. A grip unit at the first end is provided. The grip unit is adapted to grip the medical device. A shank member proximal to the second end is also provided. The shank member is adapted to engage with a drive unit that is adapted to provide a rotational force to the longitudinal member such that the gripped medical device is rotated around the member axis. The inserter includes an indicator unit adapted to provide a visual indication of number of turns of the longitudinal member when driven by the drive unit. The grip unit is adapted to releasably grip the medical device such that during installation, the grip unit grips the medical device, but when the medical device is installed, the grip unit is adapted to release the grip of the medical device.

The medical device includes an abutment for a bone anchored hearing aid and/ or a fixture for the bone anchored hearing aid. Typically, the fixture is anchored by screwing external threads of the fixture to the skull bone of the recipient. The abutment is usually detachably attached to the fixture by screwing abutment threads to internal threads of the anchored fixture. However, the abutment may directly be screwed to the bone as well.

The member axis includes an axis along a length of the inserter between the first end and the second end. The longitudinal member may be designed with a long shank member, which provides a line of sight of the work area, i.e. installation site, to the operator.

The drive unit may include a selection unit that is adapted to be set the drive unit at low speed with automatic torque control. For example, the torque is in the range of 10-50 Ncm. For a compact bone a torque in the range of 40-50 Ncm may be used whereas for a compromised or soft bone a torque in the range of 10-30 Ncm such as 10-20 Ncm is preferred. The speed may be in the range of 20-35 rpm such as between 20-30 rpm, preferably between 25-30 rpm. Using the disclosed inserter comprising the indicator unit, an operator of the inserter may easily count the number of turns of the longitudinal member, in effect the number of turns of the gripped medical device, during installation of the medical device in the recipient.

In one embodiment, the indicator unit includes a coupling portion adapted to couple with the longitudinal member of the inserter. The coupling portion may be permanently or removably coupled to the longitudinal member. The indicator unit further includes at least one arm extending outward from the coupling portion, the arm being adapted to rotate simultaneously with the longitudinal member, when the coupling portion is coupled to the longitudinal member and the longitudinal member is driven by the drive unit. The "permanently" indicates that the indicator unit is an integral part of the inserter, i.e. the indicator unit and the inserter are inseparable single unit.

It is desirable that the implant is installed perpendicular to the bone surface. When installing the implant especially while using the minimally invasive technique the bone surface is not visible, therefore during insertion, the skin surface is used as the reference plane to determine whether the implant is being positioned perpendicularly. Therefore, the indicator unit including the at least one arm is particularly useful to help the operator holding the implant/abutment inserter assembly perpendicular to the skin surface and perform rotation of the longitudinal member and the implant perpendicular to the skin/ bone surface.

In an embodiment, during installation of the medical device, the member axis is adapted to stay perpendicular to a reference skin surface and/ or bone surface by keeping the at least one arm parallel to the reference skin surface and/ or bone surface. Keeping the member axis perpendicular in this way allow for achieving alignment between the member axis and the pre-drilled hole or a fixture axis of an already installed fixture, thus avoiding or at least reducing the possibility of micro-traumata at the skin-implant interface. This is useful in both the minimally invasive surgery where only skin surface is visible during surgery and also in flap surgery where the bone surface is also visible during surgery.

In an embodiment, the removably coupled coupling portion includes a structure comprising a peripheral opening such that the peripheral opening expands under pressure allowing a coupling section (interfacing surface) of the longitudinal member to laterally slide through the expanded peripheral opening into the structure, which retracts to firmly hold on to the coupling section when the coupling section is within the structure. The structure may be visualized as hollow cylinder or a ring having an opening along it circumference and the coupling includes snap-fit joint of the indicator unit to the coupling section of the longitudinal member. The opening represents a minor arc of the periphery. The minor arc may have an angle of measure preferably of less than 90 degrees at the center of the structure.

Alternatively, in another embodiment, the removably coupled coupling portion includes a body including a through-hole allowing the longitudinal member to longitudinally slide through the hole such that an inner geometry of the hole are adapted to interact and fit against an interfacing surface of the longitudinal member.

In one embodiment, the fitting may include a friction fit between the inner geometry of the through-hole and the interfacing surface of the longitudinal member, the inner geometry including a plurality of stiff or compressible projections. In another embodiment, the inner geometry of the through-hole includes a male/ female member that is adapted to snap fit into a female/ male member provided at the interfacing surface of the longitudinal member. The interacting indicator unit male member - inserter female member or indicator unit female member - inserter male member may either include a regular shape such as like a ring or separated interacting members around the interfacing surface of the inserter and inner geometry of the indicator unit. In yet another embodiment, the inner geometry includes a first thread that is adapted to screw into a second thread provided at the interfacing surface of the longitudinal member. In yet another embodiment, the inner geometry includes a first magnetic unit that is adapted to attractively attach to a second magnetic unit of the interfacing surface of the longitudinal member. The magnetic arrangement may either be provided in a continuous form like interacting magnet rings or as discontinuous interacting magnet units. It is apparent that the attraction force between the first magnet and second magnet should be such that indicator unit does not move relative to the inserter when the inserter-indicator unit is rotated, using the drive unit, around the member axis. In yet another embodiment, the inner geometry may include at least a pair of pins that is adapted to fit into an interfacing surface including a groove having pin specific locking mechanism such as similar to a byaonet mount. It is apparent that the pair of pins may be provided in the interfacing section and the groove with the pin specific locking mechanism in the inner geometry, i.e. the inner geometry includes a groove having pin specific locking mechanism adapted to receive and lock with a pair of pins provided at the interfacing surface of the longitudinal member. Other locking mechanisms between the inner geometry and the interfacing section are also possible such as the mechanisms known from lens mounts for cameras, storz connection, etc.

In an embodiment, the indicator unit comprises a coupling portion comprising a cylindrical body. The cylindrical body comprises a through-hole that is adapted to allow the longitudinal member to longitudinally slide through the hole such that an inner geometry of the hole are adapted to interact and fit against an interfacing surface of the longitudinal member. The inner geometry comprises one full notch cut or at least one partial notch cut such as three partial notch cuts along height of the cylindrical body. The term "full" refers to if the cut is along the entire height of the cylindrical body and or "partial" refers to if the cut is only along some height of the cylindrical body. In use, when the longitudinal member slides through the hole, the full notch cut or the partial notch cut allows for expansion of the cylindrical body and fits the cylindrical body against the interfacing surface of the longitudinal member. As the notch cut(s) allows the indictor to flex and fit the longitudinal member, the same indictor unit may be used with different sizes of longitudinal members.

The coupling portion may further include at least one arm extending outward from a side section of the coupling portion. If a plurality of arms are includes, such plurality of arms may include same or partially or completely visually distinct characteristics or same visual characteristics. In an embodiment, one arm of the plurality of arms comprises visually distinct characteristics. In such a scenario, the one arm is taken in a reference arm for providing visual indication of number of turns.

In an embodiment, the coupling portion includes an extension part adapted to engage (such as rest on) with a surface of the inserter such that the at least one arm or plurality of arms are parallel to the surface, wherein the surface is perpendicular to the member axis. This arrangement ensures that the indicator unit is in level with the medical device when the medical device is gripped by the grip unit, thereby increasing the reliability of the visual indication. For example, the coupling portion includes a step arrangement comprising an extension part that extends further than the cylindrical body that comprises the inner geometry comprising one full notch cut or at least one partial notch cut. The extension part may include at least one extension (preferably a plurality of extensions) that are adapted to engage (such as rest on) with a surface of the inserter such that the at least one arm or plurality of arms are parallel to the surface, wherein the surface is perpendicular to the member axis.

In the embodiment where the engagement between the extension part and surface of the inserter occurs, the extension part comprises a first engagement element and the surface comprises a second engagement element, wherein the first engagement element and the second engagement element are adapted to cooperate for providing the engagement. The engagement allows for immovably locking the extension part relative to the longitudinal shaft. In a first embodiment, the first engagement element and second engagement element are magnets of opposite polarities. In a second element, the first engagement element is periphery of the extension part and the second engagement element is a flexible groove along a periphery of the surface, the groove being adapted to flex for receiving the periphery of the extension element. In a third embodiment, the first engagement element and the second engagement elements form complementary parts of a latch mechanism.

In an embodiment, the extension part is adapted to sandwich only between at least one arm/ the plurality of arms and the surface while the extension part is adapted to rest on the surface such that at least one arm/ plurality of arms are perpendicular to the member axis. This allows for further reducing vibrations of the arm during rotation of the inserter around the member axis.

In another embodiment, the indicator unit comprises at least one marking at a section of the longitudinal member or the at least one arm. The at least one marking is visible during installation. These marking may be a line or a visible dot or any such identifying feature that allows the operator to count the number of turns of the longitudinal member during the installation by counting the number of rotations of the at least one marking. Such marking may be created by means of laser marking or etching. This may be achieved by laser marking, or by forming a hard carbon coating such as diamond like carbon, on the part where marking is to be provided, followed by laser marking or etching. This may also be achieved by means of enamel or lacquer paint. The line solution is typically preferred over the "at least one arm" solution for flap surgery because the arms might interfere with the surrounding skin and instruments like skin retractors. However, the at least one arm may be designed (described later) such that they are usable with the flap surgery as well.

In one embodiment, the indicator unit is positioned closer to or away from the second end than to the first end. Positioning the indicator unit comprising the at least one arm closer to the second end provides a better view of the work area, i.e. the implant installation site. Whereas, positioning the indicator unit away from the second end assists in providing a visual perception to the operator whether the member axis is perpendicular to the reference skin surface surrounding the longitudinal member. This is achieved by observing and keeping the at least one arm parallel to the reference skin surface, thereby assisting in aligning the implant with the axis of predrilled hole or fixture axis. Typically, an optimum positioning of the indictor unit along the longitudinal member may be defined such that an upper connected height, i.e. from the second end to mid-point of the indicator unit is at least 2 times, such as at least 2.5 times, 3 times, 3.5 times, 4 times, 4.5 times and so on in increment of 0.25 of a lower connected height, i.e. from first end to the mid-point of the indicator unit. Other ratios are also within the scope of the invention.

In one embodiment, the at least one arm comprises a plurality of arms spread around circumference of a side section of the indicator unit. The plurality of arms may include same or partially or completely visually distinct characteristics or same visual characteristics. The "partial" refers to situations where at least two of the arms but not all arms have same visual characteristics. The "completely" refers to situations where the plurality of arms include different visual characteristics. Such visually distinct characteristics may be selected from a group consisting of different arm lengths, different arm thickness, different variation of thickness along the arm lengths, different shape (straight or curved), different angle with the member axis, different shape of a distal end of the arm such as distal end being shaped as an arrow or a diamond or circle, different arm color and a combination thereof. It might be useful to designate one of the visually distinct arms as the reference point to count the number of turns. For example, an arm having a diamond shape distal end may be designated as the reference arm.

In the preceding embodiment, the different arm color may be obtained in a number of ways. For example, in one implementation, the different color may be achieved by laser marking or etching, or by forming a hard carbon coating such as diamond like carbon, followed by laser marking or etching. In another embodiment, the different color may also be achieved by a 2-component injection molding where one member is molded from a plastic with different color than the rest of the inserter unit body. One member could also be colored by means of enamel or lacquer paint.

In an embodiment, the at least one marking comprises a plurality of markings at the at least one arm and/ or around circumferences of the longitudinal member. The plurality of markings includes same or partially or completely visually different indications or visually same indications. The "partial" refers to situations where at least two of the arms but not all arms have same visual characteristics. The "completely" refers to situations where the plurality of arms include different visual characteristics. The completely different indications is selected from a group consisting of different colors, different shades of same color or a combination thereof.

In a first embodiment, the at least one arm is perpendicular or substantially perpendicular to the member axis. This is particularly useful in providing a visual perception that the at least one arm is parallel to the skin surface, thereby assisting in aligning the implant with the axis of predrilled hole or fixture axis. In a second embodiment, the at least one arm is at an angle with the member axis such that a distal end of the at least one arm is closer to the second end than to the first end of the inserter. In a third embodiment, the at least one arm is curved such that the distal end(s) of the at least one arm is closer to the second end than to the first end of the inserter. In a fourth embodiment, the at least one arm includes at least one bend along the arm length such that along the arm length from the proximal end to the distal end, the arm gets closer to the second end than to the first end of the inserter. The recited four embodiments work well with the minimally invasive surgery. The first embodiment may work with the flap surgery, if the arm length is kept to a small value but the second, third and fourth embodiments are particularly useful for the conventional flap surgery.

In an embodiment, the indicator unit may also include a side section comprising at least one concave shaped holding groove. The concave holding groove provides a finger grip.

Typically, the medical device comprises a threaded section of a specified length comprising a predetermined screw pitch - rotation relationship, the installation comprising fastening the threaded section in the recipient of the medical device. For a fixture, the fastening is between the threaded section and skull bone of the recipient and for the abutment, the fastening is between the threaded section and the internal threads of the fixture that is already installed in the skull bone. The indicator unit is adapted to indicate number of turns of the longitudinal member when driven by the drive such that the indication represents lengthwise movement of the medical device based on the screw pitch-rotation relationship. This relationship is critical in determining number of turns needed to install the implant comprising a threaded section of a particular length. For example, for a 4 mm implant with a pitch of 0,6 mm a little over 6,6 turns is needed to get the implant fully seated against the bone. Thus, a reliable means to count the number of turns during installation would ensure a properly seated implant. Since installation typically performed with a speed of 20 to 35 rpm such as 25-30 rpm, it is possible to do such counting while using the indicator unit during installation.

In one embodiment, the at least one arm includes a smooth surface. Alternatively, the at least one arm may include a non-smooth surface. Additionally or alternatively, in another embodiment, the at least one arm includes uniform thickness along the arm length or a thickness that decreases from a proximal end that is closer to the side section to a distal end that is away from the side section. The decrease is selected from a group consisting of a gradual decrease, a step wise decrease, and a combination thereof where the decrease is gradual between subsequent steps. Having the arms with thicker section close to the side section provides enough strength and durability to the arms and reducing thickness towards the distal end prevents restricting the view of the work area.

During the indication of the number of turns, such determination/ indication is with reference to a user chosen reference point. For example, the operator may choose positioning of the at least one arm and/ or marking as the start of the rotation and this is chosen as the reference point.

In an embodiment, the indicator unit includes a secondary unit that is adapted to be positioned at the reference point on a reference surface and adapted to be immovable relative to rotation of the longitudinal member. Such secondary unit may be positioned at the skin of the patient that is adapted to receive the medical device, where the skin adjacent to the drill site acts as a reference surface. The secondary unit comprises an arm facing side and a reference surface facing side. The arm facing side includes a first signal unit and at least one of the arms of the indicator unit includes a second signal unit, wherein the first signal unit and second signal unit cooperate to provide an indication signal representing a rotation when the first signal unit passes over the second signal unit during rotation of the longitudinal member. In an embodiment, the first signal unit may include a light source such as a light emitting diode (LED) and the second signal unit includes a photodetector or vice versa such that when the first signal unit passes over the second signal unit, the photodetector senses the emitted light from the light source. In one such implementation, it is conceivable that plurality of arms comprise photodetectors having different characteristics such that the indication signals received from each photodetector is of different characteristics. This allows for determining partial rotations and not just one complete rotation. In another embodiment, the first signal unit and second signal unit form a magnetic light system whereby each time the first signal unit passes over the second signal unit, an indication signal comprising light is provided.

In an embodiment, the reference surface facing side may include an adhering element such as an adhesive that is used to adhere the secondary unit at the reference point.

In an embodiment, the indicator unit includes a processor that is adapted communicatively connected to at least one of the first signal unit and the second signal unit and adapted to receive the indication signal and interpret the indication signal in order to increase a counter by one or a fraction of one every time the indication signal is received. The counter value thus, represents the number of rotations of the longitudinal member. Additionally, the processor may also be adapted to determine the lengthwise movement of the medical device based on the screw pitch-rotation relationship as described earlier. Additionally or alternatively, the inserter may include a display unit to provide a visual indication of the number of turns and/ or determined lengthwise movement based on the counter value. Additionally or alternatively, the indication may include audible sound such as by way of beeps for example one beep for one rotation, two beeps for two rotation and so on.

In an embodiment, the indicator unit includes a processor adapted to count number of turns of the at least one arm as a function of degrees traversed with respect to a reference. The reference is set at the start of the installation process. This reference may represent a zero degree at the start of the installation process and during rotation of the longitudinal member/ indicator unit, the processor measures the degree of rotation such that at each 360 deg rotation, the number of turns count is increase by 1. The processor is adapted to indicate the rotation in partial rotations such as 0.3 rotations, 4.1 rotations, 5.6 rotations, where each 0.1 unit indicates 36 deg rotation. The processor may be adapted to indicate rotations with finer least count like 0.05 degs, etc. Additionally, the processor may also be adapted to determine the lengthwise movement of the medical device based on the screw pitch-rotation relationship as described earlier. Additionally or alternatively, the inserter may include a display unit to provide a visual indication of the number of turns and/ or determined lengthwise movement.

In yet another embodiment, the processor may be adapted to receive a threshold counter value or a threshold insertion depth. The threshold counter value corresponds to the maximum lengthwise movement of the medical device (threshold insertion depth) that is considered safe for the patient. The processor, based on the received indication signal or insertion depth estimate in accordance with the screw pitch-rotation relationship, is adapted to provide an alarm signal to warn the operator when the number of rotations corresponding to the counter value reaches the threshold counter value or lengthwise movement of the medical device reaches the threshold insertion depth.

In an embodiment, the indicator unit comprises a transmitter for transmitting the number of rotations and/ or determined lengthwise movement of the medical device to a remote unit. The remote unit may include a remote display unit and/ or a remote database and/ or a remote processing unit. The remote database may store the received data and the remote processing unit may utilize the stored data for analysis purposes.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. The individual features of each aspect may each be combined with any or all features of the other aspects. These and other aspects, features and/or technical effect will be apparent from and elucidated with reference to the illustrations described hereinafter in which:
Fig. 1 illustrates a conventionally known inserter;
Fig. 2A illustrates an indicator unit according to an embodiment of the disclosure;
Fig. 2B illustrates an indicator unit according to another embodiment of the disclosure;
Fig. 3 illustrates an inserter comprising an indicator unit according to an embodiment of the disclosure;
Fig. 4 illustrates an inserter comprising an indicator unit for a flap surgery according to an embodiment of the disclosure;
Fig. 5 illustrates an inserter comprising an indicator unit according yet another embodiment of the disclosure;
Fig. 6 illustrates a side section including a concave shaped groove according to an embodiment of the disclosure;
Fig. 7A illustrates an inserter comprising a female member according to an embodiment of the disclosure;
Fig. 7B illustrates an indicator unit comprising a male member according to an embodiment of the disclosure;
Fig. 7C illustrates a sectional view of the inner geometry comprising the male member according to an embodiment of the disclosure;
Fig. 8A illustrates an indicator unit comprising a first thread according to an embodiment of the disclosure;
Fig. 8B illustrates an inserter comprising a second thread according to an embodiment of the disclosure;
Fig. 9A illustrates an indicator unit comprising an arrow shaped distal end of the arm according to an embodiment of the disclosure;
Fig. 9B illustrates a diamond shaped distal end of the arm according to an embodiment of the disclosure
Fig. 9C illustrates a circular shaped distal end of the arm according to an embodiment of the disclosure;
Fig. 10 illustrates an indicator unit having a peripheral opening and the inserter according to an embodiment of the disclosure;
Fig. 11 illustrates an indicator unit comprising partial notch cuts according to an embodiment of the disclosure;
Fig. 12 illustrates a stepped arrangement comprising an extension part according to an embodiment of the disclosure; and
Fig. 13 illustrates an indicator unit including a secondary unit according to an embodiment of the disclosure.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practised without these specific details.

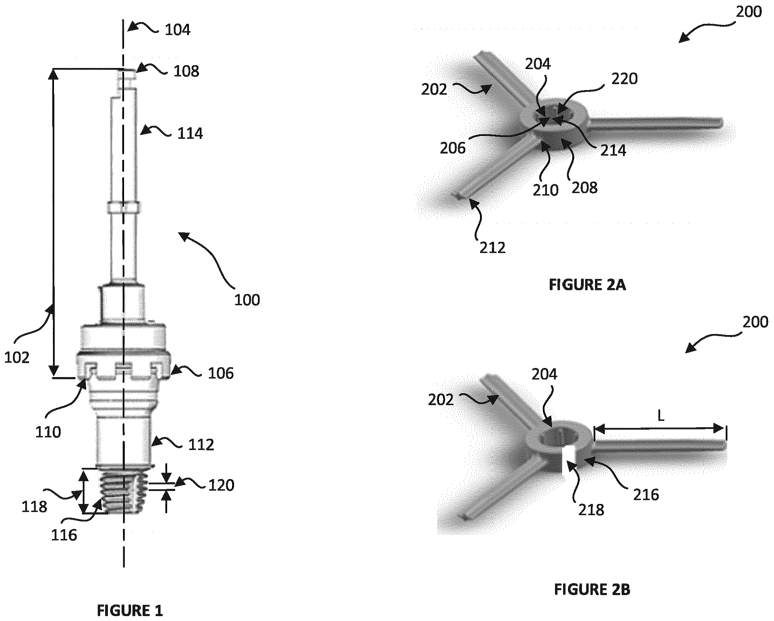

Figure 1 illustrates a conventionally known inserter. The inserter 100 is used for installing a medical device 112 via rotation in a recipient. The inserter includes a longitudinal member 102 adapted to rotate around a member axis 104. The longitudinal member 114 includes a first end 106 and a second end 108. A grip unit 110 is provided at the first end 106, the grip unit being adapted to grip the medical device 112. A shank member 114 proximal to the second end 108 is also provided. The shank member 114 is adapted to engage with a drive unit (not shown) that is adapted to provide a rotational force to the longitudinal member 102 such that the gripped medical device 112 is rotated around the member axis 104. The inserter according to an embodiment of the disclosure also includes an indicator unit (200, Fig. 2) adapted to provide a visual indication of number of turns of the longitudinal member when driven by the drive unit.

The medical device 112 such as an abutment for a bone anchored hearing aid includes a threaded section 116 of a specific known length 118 and a known pitch 120. Thus, the threaded section 116 comprising a predetermined screw pitch - rotational relationship allows for determining number of turns needed to install the implant of a particular length. For example, for a 6 mm implant with a pitch of 0,4 mm would require 15 turns to get the implant fully seated against the bone.

Fig. 2A illustrates an indicator unit according to an embodiment of the disclosure. The indicator 200 unit includes a coupling portion 204 adapted to permanently or removably couple with the longitudinal member (Fig. 1, 114) of the inserter 100. The indicator unit also includes at least one arm (represented by three arms 202) extending outward from a side section 208 of the coupling portion. The at least one arm is adapted to rotate simultaneously with the longitudinal member, when the coupling portion 204 is coupled to the longitudinal member and the longitudinal member is driven by the drive unit.

Although the at least one arm is illustrated by way of three arms, however, it would be apparent to the skilled person that the number of arms may vary such as two arms, four arms, five arms and so on. The higher number of arms may allow for more precise assessment of number of turns but it may be useful to keep the number of arms to a reasonable number such as two or four or five, preferably three - so that operator's view of the installation site is not overly restricted.

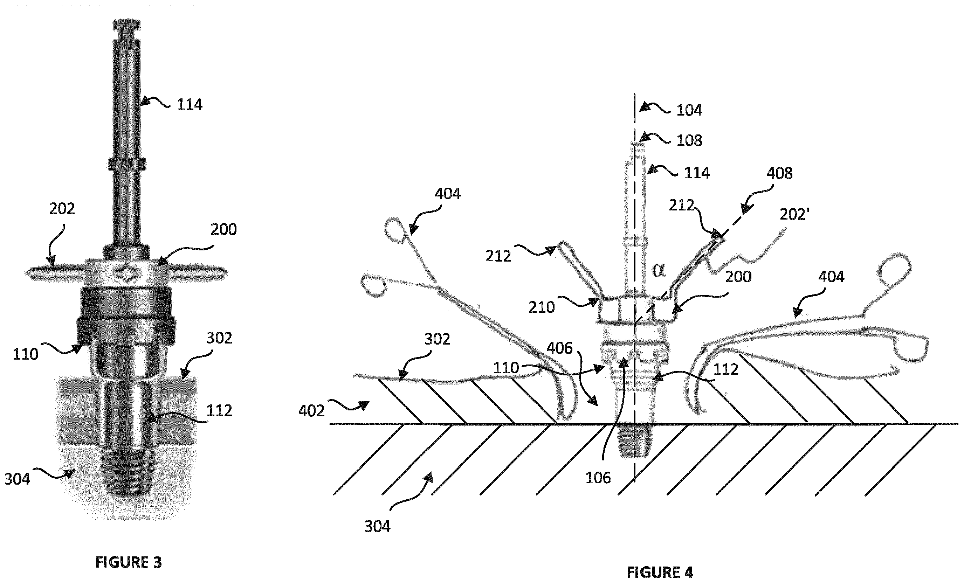

In an embodiment, as illustrated in Fig. 2A, the removably coupled coupling portion 204 includes a body comprising a through-hole 214 allowing the longitudinal member 114 to longitudinally slide through the hole 214 such that an inner geometry 206 of the hole are adapted to interact and fit against an interfacing surface of the longitudinal member (see Fig. 3).

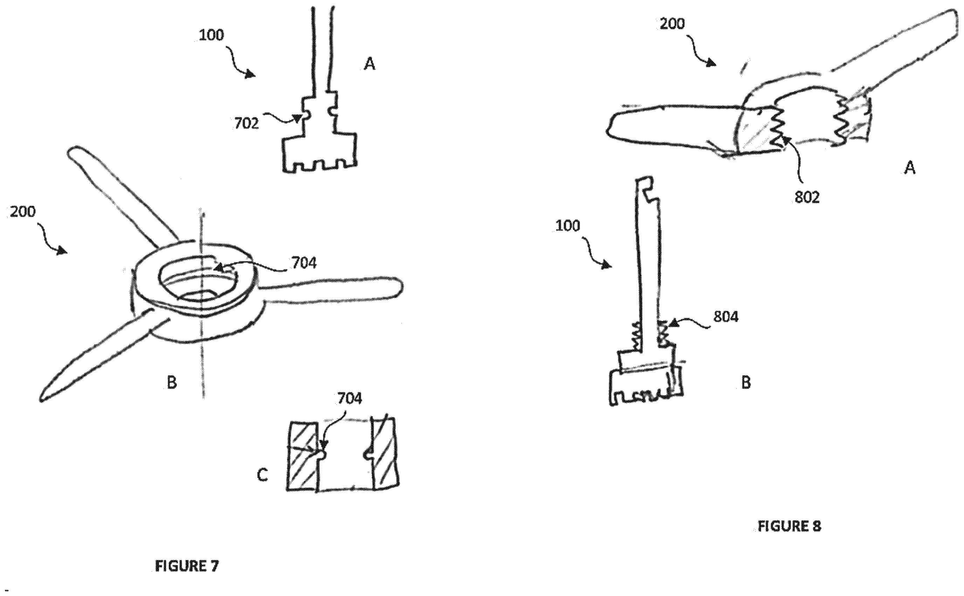

In one embodiment, the fitting may include a friction fit between the inner geometry (Fi. 2B, 206) of the through-hole and the interfacing surface of the longitudinal member, the inner geometry including a plurality of stiff or compressible projections (Fig. 2B, 220). In another embodiment, the inner geometry of the through-hole includes a male/ female member that is adapted to snap fit into a female/ male member provided at the interfacing surface of the longitudinal member. For example, Fig. 7A illustrates the inserter 100 comprising a female member 702 at the interfacing surface, Fig. 7B illustrates the indicator unit 200 comprising a male member 704 at the coupling portion. Fig. 7C illustrates a sectional view of the inner geometry comprising the male member 704. In yet another embodiment, the inner geometry includes a first thread that is adapted to screw into a second thread provided at the interfacing surface of the longitudinal member. This is illustrated in Fig. 8, wherein Fig. 8A illustrates the indicator unit 200 comprising a first thread 802 at the coupling portion and Fig. 8B illustrates an inserter 100 comprising a second thread 804 at the coupling section. In yet another embodiment, the inner geometry includes a first magnetic unit that is adapted to attractively attach to a second magnetic unit of the interfacing surface of the longitudinal member. In other different embodiments, other locking mechanisms between the inner geometry and the interfacing section are also possible such as the mechanisms known from the byaonet mount, lens mounts for cameras, storz connection, etc.

The indicator unit 200 includes a proximal end 210 that is closer to the side section 208 and a distal end 212 that is away from the side section 208. The lenght of the at least one arm is indicated as L (see Fig. 2B).

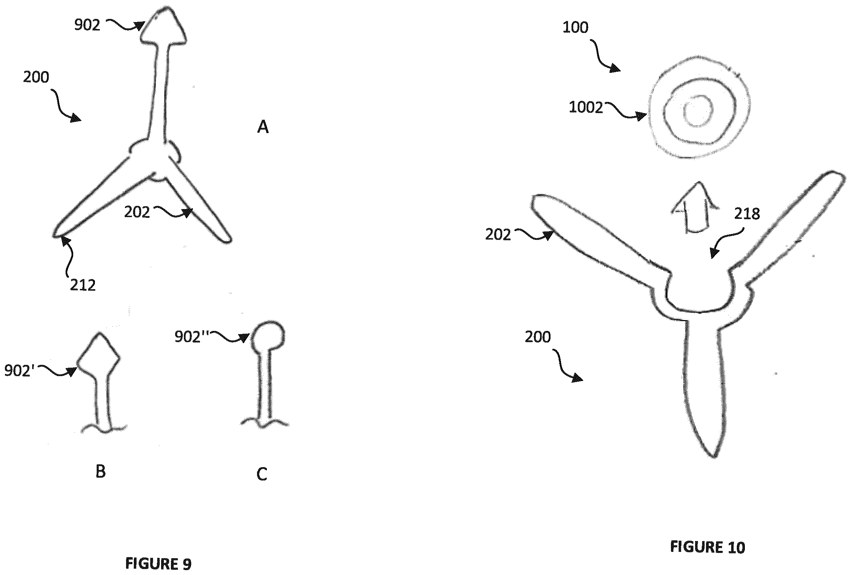

In another embodiment, as illustrated in Fig. 2B, the removably coupled coupling portion 204 includes a structure including a peripheral opening 218. The peripheral opening is provided at a periphery 216 of the side section/ coupling portion such that the peripheral opening expands under pressure. This allows a coupling section of the longitudinal member 114 to laterally slide (sidewards) through the expanded peripheral opening into the structure, which retracts to firmly hold on to the coupling section (interfacing surface) when the coupling section is within the structure. This embodiment is further represented in Fig. 10, which illustrates the indicator unit 200 comprising arms 202 comprising the peripheral opening 218 and the inserter 100 comprising the coupling section (1002, interfacing surface). As described in Fig. 2B, using the peripheral opening, the coupling section and the indicator unit are snap-fit with each other. It is apparent that the choice of the inner periphery of the structure is in relation to the dimensions of the coupling section of the longitudinal member.

Fig. 3 illustrates an inserter comprising an indicator unit according to an embodiment of the disclosure. The inserter comprising permanently or detachably attached indicator unit 200 and the medical device 112, which is inserted in a bone 304 of the recipient. During installation, the shank member 114 is engaged with the drive unit and the grip unit 110 grips the medical device. 302 represents an intact skin surface surrounding the hole and acts as the reference surface to assess alignment, the implant is installed in the hole. This is usually the situation in the minimally invasive surgery, where the bone installation site is not visible. The at least one arm 202 of the indicator unit 200 are particularly useful in providing a visual perception that the at least one arm is parallel to the skin surface, thereby assisting in aligning the implant with the axis of predrilled hole or fixture axis. In this embodiment, the at least one arm is shown as perpendicular or substantially perpendicular to the member axis. However, other embodiments of the shape and/ or orientation of the at least one arm as described earlier in the text are also useable.

Fig. 4 illustrates an inserter comprising an indicator unit 200 for a flap surgery according to an embodiment of the disclosure. In the flap surgery, the soft tissue and skin 402 is fully dissected and an installation site 406 is provided by uncovering a surface of a bone 304 to which the medical device 112 is to be anchored. The dissected skin is held in place during the surgery using a retractor 404. During installation, the shank member 114 is engaged with the drive unit and the grip unit 110 grips the medical device. 302 represents an intact skin surface surrounding the installation site. In this type of surgery, either the skin surface 302 or the exposed bone surface of the installation site 406 may be used as the reference surface to assess alignment during installation. Although the earlier recited at least one arm that is perpendicular or substantially perpendicular to the member axis may be used. However, in order to avoid the arm interfering with the skin or other instrument, preferably any one or a combination of the following embodiments may be used.
- the at least one arm 202' is at an angle α with the member axis 104 such that a distal end 212 of the at least one arm is closer to the second end 108 than to the first end 106 of the inserter. The angle α is shown between the member axis 104 and the represented arm line 408;
- the at least one arm 202' is curved along an arm length (L, see Fig. 2B) such that the distal end 212 of the at least one arm 202' is closer to the second end 108 than to the first end 106 of the inserter. In this embodiment, the curve is such that it avoids interference with the surrounding skin during surgery;
- the at least one arm 202' includes at least one bend (not shown) along the arm length (L, see Fig. 2B) such that along the arm length from a proximal end 210 to the distal end 212, the arm gets closer to the second end 108 than to the first end 106 of the inserter.

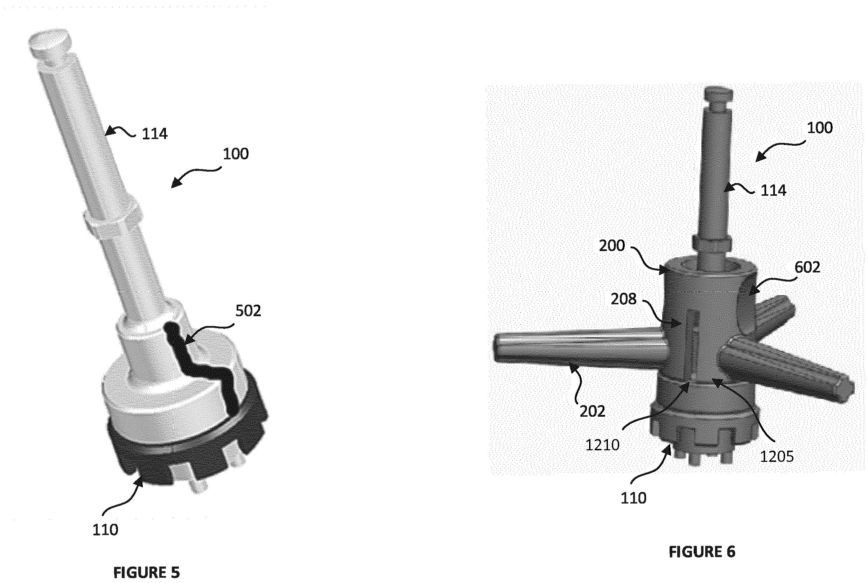

Fig. 5 illustrates an inserter comprising an indicator unit according another embodiment of the disclosure. The indicator unit 100 includes the grip unit 110 and the shank member 114 and further includes at least one marking 502 at a section of the longitudinal member (see Fig. 1, 102) or at least one arm (for example, 202, Fig. 2). The at least one marking is visible during installation of the medical device. Such marking may be created by means of laser marking or etching.

Fig. 6 illustrates a side section including a concave shaped groove according to an embodiment of the disclosure. The indicator unit 200 is shown to be coupled with the inserter 100. The inserter unit includes the side section 208 that includes at least one concave shaped holding groove 602 that may be used for gripping purposes such used as a finger grip.

In an embodiment, the at least one arm includes a smooth surface or a non-smooth surface (illustrated in Fig. 6). Additionally or alternatively, the at least one arm includes uniform thickness along the arm length L. Referring back to Fig. 2A or Fig. 6, as an alternative, thickness is such that the thickness decreases from the proximal end 210 that is closer to the side section 208 to a distal end 212 that is away from the side section 208. The decrease is selected from a group consisting of a gradual decrease, a step wise decrease, and a combination thereof where the decrease is gradual between subsequent steps.

For indicating the number of turns, such determination/ indication is with reference to a user chosen reference point. For example, the operator may choose positioning of the at least one arm and/ or marking as the start of the rotation and it is chosen as the reference point. This is usually facilitated if one of the arms of the at least one arm is visually distinct. Although different implementations are proposed earlier in the text for creating a visually distinct characteristics, but one of the more simpler embodiments includes an indicator unit comprising an indicator unit arm including a different shaped distal end. This is illustrated in different embodiments of Fig. 9, where Fig. 9A that illustrates the indicator unit 200 comprising arms 202 where one of the distal ends 212 is shaped as an arrow 902. Other embodiments include a diamond shaped distal end (902', Fig. 9B) and a circular shaped distal end (902", Fig. 9C). It would be apparent to the skilled person that other visually distinct shapes for distal end of the arm are also possible and are within the scope of this disclosure.

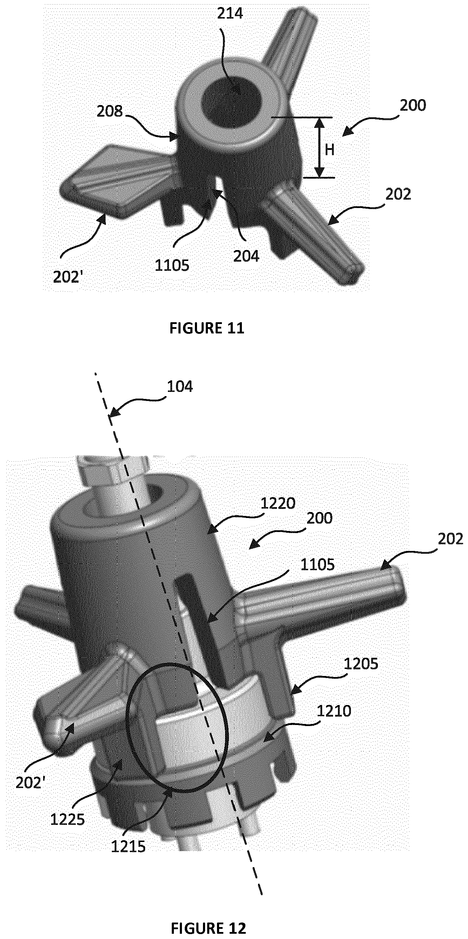

Fig. 11 illustrates an indicator unit comprising partial notch cuts according to an embodiment of the disclosure. The indicator unit 200 comprises a coupling portion 204 comprising a cylindrical body. The cylindrical body comprises a through-hole 214 that is adapted to allow the longitudinal member to longitudinally slide through the hole such that an inner geometry of the hole are adapted to interact and fit against an interfacing surface of the longitudinal member. The inner geometry comprises one full notch cut (like opening 218, Fig. 2B) or at least one partial notch cut 1105 such as three partial notch cuts along height H of the cylindrical body. The term "full" refers to if the cut is along the entire height of the cylindrical body and or "partial" refers to if the cut is only along some height of the cylindrical body. In use, when the longitudinal member slides through the hole, the full notch cut or the partial notch cut allows for expansion of the cylindrical body and fits the cylindrical body against the interfacing surface of the longitudinal member. As the notch cut(s) allows the indictor to flex and fit the longitudinal member, the same indictor unit may be used with different sizes of longitudinal members.

The coupling portion 204 may further include at least one arm 202 extending outward from a side section 208 of the coupling portion. If a plurality of arms are includes, such plurality of arms may include same or partially or completely visually distinct characteristics or same visual characteristics. In an embodiment, one arm 202' of the plurality of arms comprises visually distinct characteristics. In such a scenario, the one arm is taken as a reference arm for providing visual indication of number of turns.

Fig. 12 illustrates a stepped arrangement comprising an extension part according to an embodiment of the disclosure. The coupling portion 204 includes an extension part 1205 (also see Fig. 6, 1205) adapted to engage (such as rest on) with a surface 1210 (also see Fig. 6, 1210) of the inserter such that the at least one arm (like 202') or plurality of arms (202 and 202') are parallel to the surface 1210, wherein the surface is perpendicular to the member axis 104. This arrangement ensures that the indicator unit 200 is in level with the medical device 112 when the medical device is gripped by the grip unit, thereby increasing the reliability of the visual indication. For example, the coupling portion includes a step arrangement 1215 comprising an extension part 1205 that extends further than the cylindrical body 1220 that comprises the inner geometry comprising one full notch cut or at least one partial notch cut 1105. The extension part may include at least one extension 1225 (preferably a plurality of extensions) that are adapted to engage (such as rest on) with a surface 1205 of the inserter such that the at least one arm 202' or plurality of arms (202 and 202') are parallel to the surface, wherein the surface is perpendicular to the member axis 104.

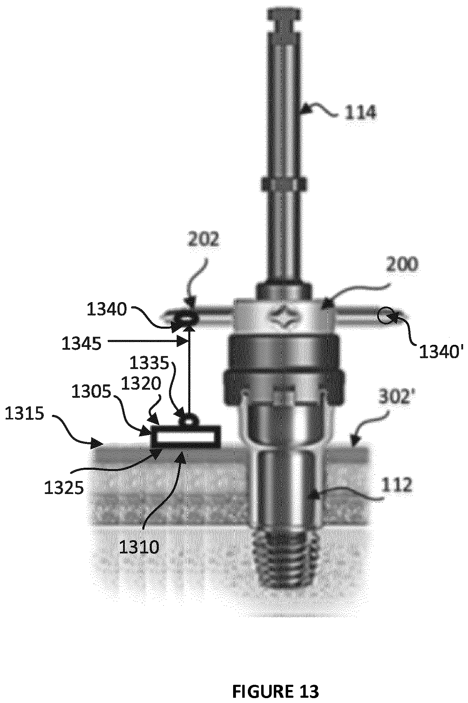

Fig. 13 illustrates an indicator unit including a secondary unit according to an embodiment of the disclosure. The indicator unit includes a secondary unit 1305 that is adapted to be positioned at the reference point 1310 on a reference surface 1315 and adapted to be immovable relative to rotation of the longitudinal member 114. Such secondary unit may be positioned at the skin 302' of the patient that is adapted to receive the medical device 112, where the skin 302' adjacent to the drill site acts as a reference surface 1315. The secondary unit comprises an arm facing side 1320 and a reference surface facing side 1325. The arm facing side includes a first signal unit 1335 and at least one of the arms 202 of the indicator unit includes a second signal unit, wherein the first signal unit and second signal unit 1340 cooperate to provide an indication signal representing a rotation when the first signal unit passes over the second signal unit during rotation of the longitudinal member. For example, the first signal unit 1335 may include a light source such as a light emitting diode (LED) and the second signal unit includes a photodetector 1340 such that when the first signal unit passes over the second signal unit, the photodetector senses the emitted light 1345 from the light source. In one such implementation, it is conceivable that plurality of arms comprise photodetectors 1340' having different characteristics such that the indication signals received from each photodetector is of different characteristics. This allows for determining partial rotations and not just one complete rotation.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

Accordingly, the scope should be judged in terms of the claims that follow.

## Claims

1. An inserter 100 for installing a medical device 112 via rotation in a recipient, the inserter 100 comprising
a longitudinal member 114 adapted to rotate around a member axis 104, the longitudinal member 114 comprising a first end 106 and a second end 108,
a grip unit 110 at the first end 106, the grip unit 110 being adapted to abut the medical device 112 while gripping the medical device 112;
a shank member 114 proximal to the second end 108, the shank member 114 being adapted to engage with a drive unit that is adapted to provide a rotational force to the longitudinal member 114 such that the gripped medical device 112 is rotated around the member axis 104; and
an indicator unit 200 comprising at least one arm 202 adapted to provide a visual indication of number of turns of the longitudinal member 114 when driven by the drive unit, wherein
during installation of the medical device, the member axis 104 is adapted to stay perpendicular to a reference skin surface 302 and/ or surface of a bone 304 by keeping the at least one arm 202 parallel to reference skin surface 302 and/ or surface of a bone 304.

2. The inserter according to claim 1, wherein the medical device 112 comprises an abutment and/ or fixture for a bone anchored hearing aid.

3. The inserter according to any of the preceding claims, wherein the indicator unit 200 comprises
a coupling portion 204 adapted to permanently or removably couple with the longitudinal member 114 of the inserter 100; and
the at least one arm 202 extending outward from a side section 208 of the coupling portion 204, the at least one arm 202 being adapted to rotate simultaneously with the longitudinal member 114, when the coupling portion 204 is coupled to the longitudinal member 114 and the longitudinal member 114 is driven by the drive unit.

4. The inserter according to any of the preceding claims, wherein the removably coupled coupling portion 204 comprises
a structure comprising a peripheral opening 218 such that the peripheral opening 218 expands under pressure allowing a coupling section of the longitudinal member 114 to laterally slide through the expanded peripheral opening 218 into the structure, which retracts to firmly hold on to the coupling section when the coupling section is within the structure; or
a body comprising a through-hole 214 allowing the longitudinal member 114 to longitudinally slide through the hole 214 such that an inner geometry 206 of the hole are adapted to interact and fit against an interfacing surface of the longitudinal member.

5. The inserter according to claim 4, wherein the inner geometry of the through-hole 214 includes
a plurality of stiff or compression projections 220 that are adapted to friction fit against the interfacing surface of the longitudinal member; or
a male/ female member 704/ 702 that is adapted to snap fit into a female/ male member 702/ 704 provided at the interfacing surface of the longitudinal member; or
a first thread 802 that is adapted to screw into a second thread 804 provided at the interfacing surface of the longitudinal member; or
a first magnetic unit that is adapted to attractively attach to a second magnetic unit of the interfacing surface of the longitudinal member; or
a pair of pins that is adapted to fit into an interfacing surface including a groove having pin specific locking mechanism or a groove having pin specific locking mechanism adapted to receive and lock with a pair of pins provided at the interfacing surface of the longitudinal member.

6. The inserter according to any of the preceding claims, wherein the indicator unit 200 comprises at least one marking 502 at a section of the longitudinal member 114 or the at least one arm 202, the at least one marking 502 being visible during installation.

7. The inserter according to any of the preceding claims, wherein the indicator unit 200 is positioned closer to or away from the second end 108 than to the first end 106.

8. The inserter according to any of the preceding claims, wherein the at least one arm 202 comprises a plurality of arms spread around circumference of the side section 208 of the indicator unit 200,
the plurality of arms 202 comprising same or partially or completely visually distinct characteristics or same visual characteristics; and
the visually distinct characteristics is selected from a group consisting of different arm lengths, different arm thickness, different variation of thickness along the arm lengths, different shape, different angle with the member axis, different shape of a distal end of the arm, different arm color and a combination thereof.

9. The inserter according to any of the preceding claims, wherein at least one marking 502 comprises a plurality of markings at the at least one arm 202 and/ or around circumferences of the longitudinal member 114,
the plurality of markings 502 comprising same or partially or completely visually different indications or visually same indications; and
the visually different indications are selected from a group consisting of different colors, different shades of same color or a combination thereof.

10. The inserter according to any of the preceding claims, wherein
the at least one arm 202 is perpendicular or substantially perpendicular to the member axis 104; or
the at least one arm 202' is at an angle α with the member axis 104 such that a distal end 212 of the at least one arm 202' is closer to the second end 108 than to the first end 106 of the inserter; or
the at least one arm 202' is curved along an arm length L such that the distal end(s) 212 of the at least one arm 202' is closer to the second end 108 than to the first end 106 of the inserter; or
the at least one arm 202' includes at least one bend along the arm length L such that along the arm length from a proximal end 210 to the distal end 212, the arm gets closer to the second end 108 than to the first end 106 of the inserter.

11. The inserter according to any of the preceding claims, wherein the indicator unit 200 comprises the side section 208 comprising at least one concave shaped holding groove 602.

12. The inserter according to any of the preceding claims, wherein
the medical device 112 comprises a threaded section 116 of a specified length 118 comprising a predetermined screw pitch - rotation relationship, the installation comprising fastening the threaded section in the recipient of the medical device, and
the indicator unit 200 is adapted to indicate number of turns of the longitudinal member 114 when driven by the drive unit such that the indication represents lengthwise movement of the medical device 112 based on the screw pitch-rotation relationship.

13. The inserter according to any of the preceding claims, wherein the at least one arm 202 comprises
a smooth surface or a non-smooth surface; and/ or
uniform thickness along the arm length L or a thickness that decreases from the proximal end 210 that is closer to the side section 208 to a distal end 212 that is away from the side section 208, the decrease is selected from a group consisting of a gradual decrease, a step wise decrease, and a combination thereof where the decrease is gradual between subsequent steps.

14. The inserter according to any of the preceding claims, wherein the indicator unit 200 comprises
a secondary unit 1305 comprises an arm facing side and a reference surface facing side, wherein the arm facing side 1320 includes a first signal unit 1335 and at least one of the arms 202 of the indicator unit includes a second signal unit 1340 and the first signal unit 1335 and second signal unit 1340 are adapted to cooperate to provide an indication signal representing a rotation when the first signal unit 1335 passes over the second signal unit 1340 during rotation of the longitudinal member 114; and/ or
a processor adapted to count number of turns of the at least one arm 202 as a function of degrees traversed with respect to a reference point 1310 that is set at the start of the insertion with or without the processor being adapted to determine the lengthwise movement of the medical device based on the screw pitch-rotation relationship; and/ or
a display unit to provide a visual indication of the number of turns and/ or determined lengthwise movement; and/ or
a transmitter for transmitting the number of rotations and/ or determined lengthwise movement of the medical device to a remote unit.

15. The inserter according to any of the preceding claims, wherein the drive unit comprises a selection unit that is adapted to set the drive unit at a speed with an automatic torque control.

## Patentansprüche

1. Inserter 100 zum Einsetzen eines Medizingeräts 112 in einen Empfänger durch Rotation, wobei der Inserter 100 Folgendes aufweist
ein Längsbauteil 114, das dazu angepasst ist, um eine Bauteilachse 104 zu rotieren, wobei das Längsbauteil 114 ein erstes Ende 106 und ein zweites Ende 108 aufweist,
eine Griffeinheit 110 an dem ersten Ende 106, wobei die Griffeinheit 110 dazu angepasst ist, bei dem Ergreifen des Medizingeräts 112 an dem Medizingerät 112 anzuliegen;
ein Schenkelbauteil 114, das proximal zu dem zweiten Ende 108 ist, wobei das Schenkelbauteil 114 dazu angepasst ist, mit einer Antriebseinheit in Eingriff zu kommen, die dazu angepasst ist, dem Längsbauteil 114 eine Rotationskraft bereitzustellen, sodass das ergriffene Medizingerät 112 um die Bauteilachse 104 rotiert wird; und
eine Anzeigeeinheit 200, die mindestens einen Arm 202 aufweist, der dazu angepasst ist, eine visuelle Anzeige der Anzahl der Umdrehungen des Längsbauteils 114 dann bereitzustellen, wenn es von der Antriebseinheit angetrieben wird, wobei
während des Einsetzens des Medizingeräts die Bauteilachse 104 dazu angepasst ist, senkrecht zu einer Referenzhautfläche 302 und/oder einer Fläche eines Knochens 304 zu bleiben, indem der mindestens eine Arm 202 parallel zu der Referenzhautfläche 302 und/oder der Knochenfläche 304 gehalten wird.

2. Inserter nach Anspruch 1, wobei das Medizingerät 112 eine Anlage und/oder eine Befestigung für ein knochenverankertes Hörgerät aufweist.

3. Inserter nach einem der vorangehenden Ansprüche, wobei die Anzeigeeinheit 200
einen Verbindungsabschnitt 204 aufweist, der dazu angepasst ist, sich dauerhaft oder trennbar mit dem Längsbauteil 114 des Inserters 100 zu verbinden; und
sich der mindestens eine Arm 202 von einem Seitenabschnitt 208 des Verbindungsabschnitts 204 nach Außen erstreckt, wobei der mindestens eine Arm 202 dazu angepasst ist, dann gleichzeitig mit dem Längsbauteil 114 zu rotieren, wenn der Verbindungsabschnitt 204 mit dem Längsbauteil 114 verbunden ist und das Längsbauteil 114 von der Antriebseinheit angetrieben wird.

4. Inserter nach einem der vorangehenden Ansprüche, wobei der trennbar verbundene Verbindungsabschnitt 204
eine Struktur, die eine Umfangsöffnung 218 aufweist, sodass sich die Umfangsöffnung 218 unter Druck ausdehnt und es einem Verbindungsabschnitt des Längsbauteils 114 ermöglicht, lateral durch die ausgedehnte Umfangsöffnung 218 in die Struktur zu gleiten, die sich zurückzieht um den Verbindungsabschnitt dann festzuhalten, wenn sich der Verbindungsabschnitt innerhalb der Struktur befindet; oder
einen Körper aufweist, der ein Durchgangsloch 214 aufweist, das es dem Längsbauteil 114 ermöglicht, in Längsrichtung durch das Loch 214 zu gleiten, sodass eine Innengeometrie 206 des Lochs dazu angepasst ist, mit einer Schnittstellenfläche des Längsbauteils wechselzuwirken und an sie zu passen.

5. Inserter nach Anspruch 4, wobei die Innengeometrie des Durchgangslochs 214 Folgendes hat
eine Vielzahl steifer oder zusammendrückbarer Vorsprünge 220, die dazu angepasst sind, reibschlüssig gegen die Schnittstellenfläche des Längsbauteils zu passen; oder
ein männliches/weibliches Bauteil 704/702, das dazu angepasst ist, in ein weibliches/männliches Bauteil 702/704 einzurasten, das an der Schnittstellenfläche des Längsbauteils bereitgestellt ist; oder
ein erstes Gewinde 802, das dazu angepasst ist, in ein zweites Gewinde 804 zu schrauben, das an der Schnittstellenfläche des Längsbauteils bereitgestellt ist; oder
eine erste magnetische Einheit, die dazu angepasst ist, sich durch Anziehung an eine zweite magnetische Einheit der Schnittstellenfläche des Längsbauteils festzumachen; oder
ein Paar Zapfen, das dazu angepasst ist, in eine Schnittstellenfläche mit einer Nut, die einen zapfenspezifischen Sperrmechanismus hat, zu passen, oder eine Nut, die einen zapfenspezifischen Sperrmechanismus hat, der dazu angepasst ist, ein Paar Zapfen zu empfangen und zu sperren, das an der Schnittstellenfläche des Längsbauteils bereitgestellt ist.

6. Inserter nach einem der vorangehenden Ansprüche, wobei die Anzeigeeinheit 200 mindestens eine Markierung 502 an einem Abschnitt des Längsbauteils 114 oder des mindestens einen Armes 202 aufweist, wobei die mindestens eine Markierung 502 während des Einsetzens sichtbar ist.

7. Inserter nach einem der vorangehenden Ansprüche, wobei die Anzeigeeinheit 200 näher an oder weg von dem zweiten Ende 108 als an dem ersten Ende 106 angeordnet ist.

8. Inserter nach einem der vorangehenden Ansprüche, wobei der mindestens eine Arm 202 eine Vielzahl von Armen aufweist, die um den Umfang des Seitenabschnitts 208 der Anzeigeeinheit 200 verteilt sind,
die Vielzahl von Armen 202 gleiche oder teilweise oder vollständig visuell verschiedene Charakteristik oder gleiche visuelle Charakteristik aufweisen; und
die visuell verschiedene Charakteristik aus einer Gruppe ausgewählt wird, die aus unterschiedlichen Armlängen, unterschiedlichen Armstärken, unterschiedlicher Variation der Stärke entlang der Armlänge, unterschiedlicher Form, unterschiedlichem Winkel zu der Bauteilachse, unterschiedlicher Form eines distalen Endes des Arms, unterschiedlicher Armfarbe und einer Kombination davon besteht.

9. Inserter nach einem der vorangehenden Ansprüche, wobei mindestens eine Markierung 502 eine Vielzahl Markierungen an dem mindestens einen Arm 202 und/oder um Umfänge des Längsbauteils 114 aufweist,
wobei die Vielzahl der Markierungen 502 gleiche oder teilweise oder vollständig visuell unterschiedliche Anzeigen oder visuell gleiche Anzeigen aufweisen; und
die visuell unterschiedlichen Anzeigen aus einer Gruppe ausgewählt werden, die aus unterschiedlichen Farben, unterschiedlichen Farbtönen der gleichen Farbe oder einer Kombination davon besteht.

10. Inserter nach einem der vorangehenden Ansprüche, wobei
der mindestens eine Arm 202 senkrecht oder im Wesentlichen senkrecht zu der Bauteilachse 104 ist; oder
der mindestens eine Arm 202' in einem Winkel α zu der Bauteilachse 104 ist, sodass ein distales Ende 212 des mindestens einen Armes 202' näher an dem zweiten Ende 108 als an dem ersten Ende 106 des Inserters ist; oder
der mindestens eine Arm 202' gekrümmt entlang einer Armlänge L ist, sodass das/die distale(n) Ende(n) 212 des mindestens einen Armes 202' näher an dem zweiten Ende 108 als an dem ersten Ende 106 des Inserters ist; oder
der mindestens eine Arm 202' mindestens eine Biegung entlang der Armlänge L hat, sodass der Arm entlang der Armlänge von dem proximalen Ende 210 zu dem distalen Ende 212 näher an das zweite Ende 108 als an das erste Ende 106 des Inserters hinkommt.

11. Inserter nach einem der vorangehenden Ansprüche, wobei die Anzeigeeinheit 200 den Seitenabschnitt 208 aufweist, der mindestens eine konkavförmige Haltenut 602 aufweist.

12. Inserter nach einem der vorangehenden Ansprüche, wobei
das Medizingerät 112 einen Gewindeabschnitt 116 von einer bestimmten Länge 118 aufweist, der eine vorbestimmte Gewindesteigungs-Rotations-Beziehung aufweist, wobei das Einsetzen das Befestigen des Gewindeabschnitts in dem Empfänger des Medizingeräts umfasst, und
die Anzeigeeinheit 200 dazu angepasst ist, eine visuelle Anzahl der Umdrehungen des Längsbauteils 114 dann anzuzeigen, wenn es von der Antriebseinheit angetrieben wird, sodass die Anzeige die Längsbewegung des Medizingeräts 112 auf Grundlage der Gewindesteigungs-Rotations-Beziehung abbildet.

13. Inserter nach einem der vorangehenden Ansprüche, wobei der mindestens eine Arm 202 Folgendes aufweist
eine glatte Fläche oder eine nichtglatte Fläche; und/oder
eine einheitliche Stärke entlang der Armlänge L oder eine Stärke, die von dem proximalen Ende 210, das näher an dem Seitenabschnitt 208 ist, zu einem distalen Ende 212 hin abnimmt, das fern von dem Seitenabschnitt 208 ist, wobei die Abnahme aus einer Gruppe ausgewählt ist, die aus einer allmählichen Abnahme, einer schrittweisen Abnahme, und einer Kombination davon besteht, wobei die Abnahme zwischen aufeinanderfolgenden Schritten allmählich ist.

14. Inserter nach einem der vorangehenden Ansprüche, wobei die Anzeigeeinheit 200 Folgendes aufweist
eine Sekundäreinheit 1305, die eine armzugewandte Seite und eine referenzflächenzugewandte Seite aufweist, wobei die armzugewandte Seite 1320 eine erste Signaleinheit 1335 hat und mindestens einer der Arme 202 der Anzeigeeinheit eine zweite Signaleinheit 1340 hat und die erste Signaleinheit 1335 und die zweite Signaleinheit 1340 zur Zusammenwirkung angepasst sind, um ein Anzeigesignal, das eine Rotation abbildet, dann bereitzustellen, wenn die erste Signaleinheit 1335 die zweite Signaleinheit 1340 während der Rotation des Längsbauteils 114 überfährt; und/oder
einen Prozessor, der dazu angepasst ist, die Anzahl der Umdrehungen des mindestens einen Armes 202 als Funktion der mit Bezug auf einen Referenzpunkt 1310 verfahrenen Grade zu zählen, der zu Beginn des Einsetzens gesetzt wird, mit oder ohne eine Anpassung des Prozessors zur Bestimmung der Längsbewegung des Medizingeräts auf Grundlage der Gewindesteigungs-Rotations-Beziehung; und/oder
eine Darstellungseinheit zum Bereitstellen einer visuellen Anzeige der Anzahl der Umdrehungen und/oder der bestimmten Längsbewegung; und/oder
ein Übertragungsgerät zum Übertragen der Anzahl der Rotationen und/oder der bestimmten Längsbewegung des Medizingeräts an eine Ferneinheit.

15. Inserter nach einem der vorangehenden Ansprüche, wobei die Antriebseinheit eine Auswahleinheit aufweist, die dazu angepasst ist, die Antriebseinheit durch eine automatische Drehmoment-Steuerung auf eine Geschwindigkeit zu setzen.

## Revendications

1. Dispositif d'insertion 100 pour l'installation d'un dispositif médical 112 par rotation dans un récipient, le dispositif d'insertion 100 comprenant
un longeron 114 conçu pour être mis en rotation autour d'un axe d'élément 104, le longeron 114 comprenant une première extrémité 106 et une seconde extrémité 108,
une unité de préhension 110 au niveau de la première extrémité 106, l'unité de préhension 110 étant conçue pour venir en butée contre le dispositif médical 112 tout en saisissant le dispositif médical 112 ;
un élément de tige 114 à proximité de la seconde extrémité 108, l'élément de tige 114 étant conçu pour venir en prise avec une unité d'entraînement qui est conçue pour fournir une force de rotation au longeron 114 de sorte que le dispositif médical saisi 112 soit entraîné en rotation autour de l'axe d'élément 104 ; et
une unité d'indication 200 comprenant au moins un bras 202 conçu pour fournir une indication visuelle du nombre de tours du longeron 114 lorsqu'il est entraîné par l'unité d'entraînement, où,
lors de l'installation du dispositif médical, l'axe d'élément 104 est conçu pour rester perpendiculaire à une surface de référence de peau 302 et/ou d'un os 304 en maintenant l'au moins un bras 202 parallèle à la surface de référence de peau 302 et/ou d'un os 304.

2. Dispositif d'insertion selon la revendication 1, où le dispositif médical 112 comprend une butée et/ou une fixation pour une aide auditive à ancrage osseux.

3. Dispositif d'insertion selon l'une quelconque des revendications précédentes, où l'unité d'indication 200 comprend
une partie de couplage 204 conçue pour se coupler de manière permanente ou amovible au longeron 114 du dispositif d'insertion 100 ; et
l'au moins un bras 202 s'étendant vers l'extérieur depuis une section latérale 208 de la partie de couplage 204, l'au moins un bras 202 étant conçu pour être mis en rotation simultanément avec le longeron 114, lorsque la partie de couplage 204 est couplée au longeron 114 et que le longeron 114 est entraîné par l'unité d'entraînement.

4. Dispositif d'insertion selon l'une quelconque des revendications précédentes, où la partie de couplage couplée de manière amovible 204 comprend
une structure comprenant une ouverture périphérique 218 de sorte que l'ouverture périphérique 218 s'agrandit sous la pression, permettant à une section de couplage du longeron 114 de glisser latéralement à travers l'ouverture périphérique 218 agrandie à l'intérieur de la structure, qui se rétracte pour tenir fermement la section de couplage lorsque la section de couplage est à l'intérieur de la structure ; ou
un corps comprenant un trou traversant 214 permettant au longeron 114 de coulisser longitudinalement à travers le trou 214 de telle sorte qu'une géométrie interne 206 du trou soit conçue pour interagir avec et à s'adapter contre une surface d'interface du longeron.

5. Dispositif d'insertion selon la revendication 4, où la géométrie interne du trou traversant 214 comprend
une pluralité de saillies rigides ou de compression 220 conçues pour s'ajuster par friction contre la surface d'interface du longeron ; ou
un élément mâle/femelle 704/702 qui est conçu pour s'encliqueter dans un élément femelle/mâle 702/704 prévu au niveau de la surface d'interface du longeron ; ou
un premier filetage 802 qui est conçu pour se visser dans un second filetage 804 prévu au niveau de la surface d'interface du longeron ; ou
une première unité magnétique qui est conçue pour se fixer par attirance à une seconde unité magnétique de la surface d'interface du longeron ; ou
une paire de goupilles qui est conçue pour s'adapter à l'intérieur d'une surface d'interface comprenant une rainure ayant un mécanisme de verrouillage spécifique à goupille ou une rainure ayant un mécanisme de verrouillage spécifique à goupille conçu pour recevoir et verrouiller avec une paire de goupilles prévues à la surface d'interface du longeron.

6. Dispositif d'insertion selon l'une quelconque des revendications précédentes, où l'unité d'indication 200 comprend au moins un marquage 502 au niveau d'une section du longeron 114 ou de l'au moins un bras 202, l'au moins un marquage 502 étant visible pendant l'installation.

7. Dispositif d'insertion selon l'une quelconque des revendications précédentes, où l'unité d'indication 200 est positionnée plus près ou plus loin de la seconde extrémité 108 que de la première extrémité 106.

8. Dispositif d'insertion selon l'une quelconque des revendications précédentes, où l'au moins un bras 202 comprend une pluralité de bras répartis autour de la circonférence de la section latérale 208 de l'unité d'indication 200,
la pluralité de bras 202 comprenant des caractéristiques identiques ou partiellement ou complètement distinctes visuellement ou des caractéristiques visuelles identiques ; et
les caractéristiques visuellement distinctes sont sélectionnées dans un groupe constitué de différentes longueurs de bras, différentes épaisseurs de bras, différentes variations d'épaisseur le long du bras, différentes formes, différents angles avec l'axe d'élément, différentes formes d'une extrémité distale du bras, différents bras de couleur et une combinaison de ceux-ci.

9. Dispositif d'insertion selon l'une quelconque des revendications précédentes, où au moins un marquage 502 comprend une pluralité de marquages au niveau de l'au moins un bras 202 et/ou autour de circonférences du longeron 114,
la pluralité de marquages 502 comprenant des indications visuellement identiques ou partiellement ou totalement différentes, ou des indications visuellement identiques ; et
les indications visuellement différentes sont sélectionnées dans un groupe constitué de différentes couleurs, de différentes nuances de la même couleur ou d'une combinaison de celles-ci.

10. Dispositif d'insertion selon l'une quelconque des revendications précédentes, où
l'au moins un bras 202 est perpendiculaire ou sensiblement perpendiculaire à l'axe d'élément 104; ou
l'au moins un bras 202' forme un angle α avec l'axe d'élément 104 de sorte qu'une extrémité distale 212 de l'au moins un bras 202' soit plus proche de la seconde extrémité 108 que de la première extrémité 106 du dispositif d'insertion ; ou
l'au moins un bras 202' est courbé sur une longueur de bras L de sorte que la ou les extrémités distales 212 de l'au moins un bras 202' soient plus proches de la seconde extrémité 108 que de la première extrémité 106 du dispositif d'insertion ; ou
l'au moins un bras 202' comprend au moins un coude le long de la longueur du bras L de sorte que, le long de la longueur du bras, depuis une extrémité proximale 210 jusqu'à l'extrémité distale 212, le bras est plus proche de la seconde extrémité 108 que de la première extrémité 106 du dispositif d'insertion.

11. Dispositif d'insertion selon l'une quelconque des revendications précédentes, où l'unité d'indication 200 comprend la section latérale 208 comprenant au moins une rainure de tenue de forme concave 602.

12. Dispositif d'insertion selon l'une quelconque des revendications précédentes, où
le dispositif médical 112 comprend une section filetée 116 d'une longueur spécifiée 118 comprenant une relation pas de vis prédéterminé - rotation, l'installation comprenant une fixation de la section filetée dans le récipient du dispositif médical, et
l'unité d'indication 200 est conçue pour indiquer le nombre de tours du longeron 114 lorsqu'elle est entraînée par l'unité d'entraînement de sorte que l'indication représente un mouvement longitudinal du dispositif médical 112 sur la base de la relation pas de vis -rotation.

13. Dispositif d'insertion selon l'une quelconque des revendications précédentes, où l'au moins un bras 202 comprend
une surface lisse ou une surface non lisse ; et/ou
une épaisseur uniforme le long de la longueur du bras L ou une épaisseur diminuant à partir de l'extrémité proximale 210 qui est plus proche de la section latérale 208 jusqu'à une extrémité distale 212 qui est éloignée de la section latérale 208, la diminution étant sélectionnée dans un groupe constitué d'une diminution progressive, d'une diminution par pas, et une combinaison de celles-ci où la diminution est progressive entre des pas successifs.

14. Dispositif d'insertion selon l'une quelconque des revendications précédentes, où l'unité d'indication 200 comprend
une unité secondaire 1305 comprenant un bras orienté latéralement et une surface de référence orientée latéralement, où le bras orienté latéralement 1320 comprend une première unité de signal 1335 et au moins un des bras 202 de l'unité d'indication comprend une seconde unité de signal 1340 et les première unité de signal 1335 et seconde unité de signal 1340 sont conçues pour coopérer afin de fournir un signal d'indication représentant une rotation lorsque la première unité de signal 1335 passe au-dessus de la seconde unité de signal 1340 pendant une rotation du longeron 114 ; et/ou
un processeur conçu pour compter le nombre de tours de l'au moins un bras 202 en fonction des degrés traversés par rapport à un point de référence 1310 qui est défini au début de l'insertion avec ou sans le processeur conçu pour déterminer le mouvement longitudinal du dispositif médical sur la base de la relation pas de vis - rotation ; et/ou
une unité d'affichage pour fournir une indication visuelle du nombre de tours et/ou d'un mouvement déterminé longitudinal ; et/ou
un transmetteur pour transmettre le nombre de rotations et/ou le mouvement longitudinal déterminé du dispositif médical à une unité distante.

15. Dispositif d'insertion selon l'une quelconque des revendications précédentes, où l'unité d'entraînement comprend une unité de sélection qui est conçue pour régler l'unité d'entraînement à certaine une vitesse avec une commande de couple automatique.
